# EUROPÄISCHE PATENTSCHRIFT

(11) **EP 3 137 044 B1**
(45) Veröffentlichungstag und Bekanntmachung des Hinweises auf die Patenterteilung: **18.09.2019**
(21) Anmeldenummer: 15716491.4
(22) Anmeldetag: 10.04.2015
(51) Int. Cl.: A61K 8/24, A61K 8/36, A61K 8/362, A61K 8/365, A61K 8/55, A61Q 17/04

(54) **SONNENSCHUTZMITTEL MIT REDUZIERTER NEIGUNG ZUR TEXTILVERFLECKUNG III**
SUNSCREEN HAVING REDUCED TENDENCY TO STAIN TEXTILES III
PRODUIT DE PROTECTION SOLAIRE AYANT UNE TENDANCE RÉDUITE À FORMER DES TACHES SUR LES TEXTILES III

(30) Priorität: 28.04.2014 DE 102014207935
(43) Veröffentlichungstag der Anmeldung: 08.03.2017
(60) Teilanmeldung: 18170090.7
(73) Patentinhaber: Beiersdorf AG, 20253 Hamburg (DE)
(72) Erfinder: WEINERT, Katrin, 22763 Hamburg (DE); BORCHERS, Kathrin, 21614 Buxtehude (DE); SCHADE, Tatjana, 25866 Mildstedt/Rosendahl (DE); BLECKMANN, Andreas, 22926 Ahrensburg (DE)
(86) Internationale Anmeldenummer: PCT/EP2015/057818
(87) Internationale Veröffentlichungsnummer: WO 2015/165715

(56) Entgegenhaltungen:
- WO-A1-2011/101250
- US-A1- 2012 156 149
- US-A1- 2013 071 341
- US-A1- 2013 104 319
- US-A1- 2013 309 185

## Beschreibung

Die vorliegende Erfindung betrifft Verfahren und Verwendungen zur Erleichterung der Auswaschbarkeit von UV-Lichtschutzfilter enthaltenden kosmetischen Zubereitungen aus Textilien, dadurch gekennzeichnet, dass dem Kosmetikum ein oder mehrere Komplexbildner zugesetzt werden.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der inhaltsstoffe, insbesondere auf hellen Textilien Flecken und Verfärbungen. Diese Verfärbungen werden insbesondere durch nicht-wasserlösliche UVA- und Die Verfleskungen sind durch Waschen mit herkömmlichen Waschmitteln kaum zu entfernen und verstärken sich während des Waschprozesses durch Wechselwirkungen mit Ionen desWaschwassers sogar noch.

Es war daher die Aufgabe der vorllegenden Erfindung, die Nachteile des Standes der Technik zu beseitigen und eine kosmetische Zubereitung (insbesondere ein Sonnenschutzmittel) enthaltend nicht-wasserlösliche UV-A-Filter und/oder Breitbandfilter zu antwickeln, welche sich leichter aus den mit der Zubereltung kontaminierten Textilen herauswaschen lassen.

Überraschend gelöst wird die Aufgabe durch ein Verfahren zur Erleichterung der Auswaschbarkeit von UV-Lichtschutzfilter enthaltenden kosmetischen Zubereitungen aus Textilien, wobei dem Kosmetikum ein oder mehrere Komplexbildner aus der Gruppe
- 1-Hydroxyethan-(1,1-diphosphonsäure)/ HEDP
- Aminotrimethylenphosphonsäure/ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Natriumpolyphosphat
- Tetranatriumpyrophosphat
und/oder deren Alkalisatze zugesetzt werden, dadurch gekennzeichnet, dass die Zubereltung 0,1 bis 3,0 Gewichts-%, bezogen auf das Gesamtgewicht der Zubereitung, an Komplexbildnern enthält,

Überraschend gelöst wird die Aufgabe durch ein Verfahren zur Reduzierung der durch UV-Lichtschutzfilter enthaltenden kosmetischen Zubereitungen hervorgerufenen Textilverfleckung, wobei dem Kosmetikum ein oder mehrere Komplexbildner aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/DTPMP
- Ethylendiamintetra(methylenphosphonsäure/ EDTMP
- Phosphonobutan-tricarbonsäure/PBTC
- Natriumpolyphosphat
- Tetranatriumpyrophosphat
und/oder deren Alkalisalze zugesetzt werden, dadurch gekennzeichnet, dass die Zubereltung 0,1 bis 3,0 Gewichts-%, bezogen auf das Gesamtgewicht der Zubereitung, an Komplexbildnern enthält.

Überraschend gelöst wird die Aufgabe durch die Verwendung von Komplexbildnern aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Natriumpolyphosphat
- Tetranatriumpyrophosphat
und/oder deren Alkalisalze,
in UV-Lichtschutzfilter enthaltenden kosmetischen Zubereitungen zur Erleichterung der Auswaschbarkeit der UV-Lichtschutzfilter aus mit den Zubereitungen Kontaminierten Textilien,dadurch gekennzeichnet, dass die Zubereitung 0,1 bis 3,0 Gewichts-%, bezogen auf das Gesamtgewicht der Zubereitung, an Komplexbildnern enthält.

Überraschend gelöst wird die Aufgabe durch die Verwendung von Komplexbildnern aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra:(methylenphosphonsäure/ EDTMP
- Phosphonobutan-tricarbonsäure/ PBTC
- Natriumpolyphosphat
- Tetranatriumpyrophosphat
und/oder deren Alkalisalze
in UV-Lichtschutzfilter enthaltenten kosmetischen Zubereitungen zur Reduzierung der durch die Zubereitung hervorgerufene Textilverfleckung, dadurch gekennzeichnet, dass die Zubereltung 0,1bis 3,0 Gewichts-%, bezogen auf das Gesamtgewicht der Zubereitung, an Komplexbildnern enthält.

Zwar kennt der Fachmann die WO2011/101250, doch konnte diese Schrift nicht den Weg zur vorliegenden Erfindung weisen.

Ferner kennt der Stand der Technik die WO 2011/101250, US 2013/007341, US 2012/156149, US 2013/309185 und US 2013/104319, die ebenfalls nicht den Weg zur vorliegenden Erfindung weisen konnten.

Die Begriffe "erfindungsgemäße Zubereitung", "erfindungsgemäß" etc, beziehen sich im Rahmen der vorliegenden Offenbarung immer auf das erfindungsgemäße Verfahren und die erfindungsgemäße Verwendung.

Erfindungsgemäß besonders bevorzugte Ausführungsformen der vorliegendenErfindung sind dadurch gekennzeiehnet, dass als Komplexbildner eine oder mehrere der Verbindungen aus der Gruppe
- Dlethylentriaminpenta(methylenphosphonsäure)/DTPMP
- Aminotrimethylenphosphonsäure/ATMP
- Ethylendiamintetra(methylenphosphonsäure/ EDTMP
- Natriumpolyphosphat
- Tetranatriumpyrophosphat
   und/oder deren Alkalisalze und/ oder deren Amin-N-Oxide eingesetzt werden.

Vorteilhaft im Sinne der vorliegenden Erfindung ist es, wenn die kosmetische Zubereitung einen oder mehrere UV-Filter gewählt aus der Gruppe der Verbindungen 4-(tert.-Butyl)-4'-methoxydibenzoylmethan (INCI Butyl Methoxydibenzoylmethane), Hexyl 2-[4-(diethylamino)-2-hydroxybenzoyl]benzoate (INCI Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält. Diese UV-Filter lassen sich nach dem Stand der Technik besonders schwer aus Textilien herauswaschen und führen zu besonders starken Verfärbungen. Die Vorteile des erfindungsgemäßen Verfahrens und der erfindungsgemäßen Verwendung treten daher bei diesen UV-Filtern besonders zu Tage.

Es ist in diesen Fällen bevorzugt, wenn das Gewichtsverhältnis der Gesamtmenge aus 4-(tert.-Butyl)-4'-methoxydibenzoylmethan (INCI Butyl Methoxydibenzoylmethane), INCI Butyl Methoxydibenzoylmethane), Hexyl 2-[4-(diethylamino)-2-hydroxybenzoyl]benzoate (INCI Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) zur Gesamtmenge an Komplexbildnern von 5:1 bis 5:3 beträgt (wobei die Gesamtmenge der Einzelmenge entspricht, wenn nur eine der Komponenten eingesetzt wird).

Die erfindungsgemäßen Verfahren oder Verwendungen sind erfindungsgemäß vorteilhaft dadurch gekennzeichnet, dass die kosmetische Zubereitung einen oder mehrere UV-Filter enthält , die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-meth-oxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist erfindungsgemäß vorteilhaft, wenn die erfindungsgemäße Zubereitung in Form einer Öl-in -Wasser-Emulsion (O/W-Emulsion) vorliegt. In einem solchen Falle ist es erfindugnsgemäß bevorzugt, wenn die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat, Natriumstearoylglutamat, Polyglyceryl-3-methylglucosedistearat, Stearinsäure, Kaliumcetylphosphat, enthält.

Vorteilhafte Ausführungsformen der vorliegenden Erfindung sind dadurch gekennzeichnet, dass die Zubereitung Acrylat/C10-30 Alkylacrylat-Crosspolymer enthält.

In einem solchen Falle ist es erfindungsgemäß bevorzugt, wenn die Zubereitung Acrylat/C10-30 Alkylacrylat-Crosspolymer in einer Menge von 0,01 bis 2,0 Gewichts-%, bezogen auf das Gesamtgewicht der Zubereitung, enthält.

Vorteilhafte Ausführungsformen der vorliegenden Erfindung sind ferner dadurch gekennzeichnet, dass die Zubereitung Vinylpyrrolidon/Hexadecen Copolymer enthält.

In einem solchen Falle ist es erfindungsgemäß bevorzugt, wenn die Zubereitung Vinylpyrrolidon/Hexadecen Copolymer in einer Menge von 0,01 bis 3,0 Gewichts-%, bezogen auf das Gesamtgewicht der Zubereitung, enthält.

Es ist erfindungsgemäß von Vorteil, wenn die Zubereitung Dialkylcarbonat, Dialkyladipat und/oder Dialkylglutarat enthält.

Dabei sind die erfindungsgemäß bevorzugten Ausführungsformen dadurch gekennzeichnet, dass die Zubereitung Di-n-Octylcarbonat (INCI Dicaprylyl Carbonate) und/oder Di-n-Butyladipat (INCI Dibutyl Adipate) enthält.

In einem solchen Falle ist es erfindungsgemäß besonders vorteilhaft, wenn die Gesamtmenge an n-Octylcarbonat (INCI Dicaprylyl Carbonate) und Di-n-Butyladipat (INCI Dibutyl Adipate) von 0,01 bis 20 Gewichts-%, bezogen auf das Gesamtgewicht der Zubereitung, beträgt (wobei die Gesamtmenge der Einzelmenge entspricht, wenn nur eine der beiden Komponenten eingesetzt werden).

Die erfindungsgemäße Zubereitung kann darüber hinaus die üblichen Inhaltstoffe enthalten und wie eine übliche Zubereitung zusammengesetzt sein.

### Vergleichsversuch

Mit dem folgenden Versuch konnte der erfindungsgemäße Effekt beispielhaft belegt werden: Es wurde jeweils 1% der erfindungsgemäßen Hilfsstoffe zu einer 4-(tert.-Butyl)-4'-methoxydibenzoylmethan (INCI Butyl Methoxydibenzoylmethane) enthaltenden Formulierung zugesetzt und die Verfleckungsreduzierende Wirkung (Reduktion b*) im Vergleich zu einer Formulierung ohne erfindungsgemäße Komplexbildner mittels beschriebener Methode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in der Abbildung 1 und Tabelle 1 dargestellt sind.
Es wurden verschiedene Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 25 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.
Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.
Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.
Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, 10 Metallkugeln als Beiladung) und im Anschluss ein Spülvorgang (20°C, 15min, Leitungswasser).
Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.
Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: getestete Zubereitungen und deren Gelbwert-Reduktion von Flecken; b*-Wert [%]**

| **INCI** | **Beispiel [%]** | |
|---|---|---|
| | **1** | **2** |
| Diethylentriam inpentamethylenphosphonsäure | | 1,00 |
| Tetrasodium Iminodisuccinate | 0,60 | 0,60 |
| Trisodium EDTA | 0,30 | 0,30 |
| Cetearyl Alcohol | 0,50 | 0,50 |
| Octyldodecanol | 2,00 | 2,00 |
| Caprylic/Capric Triglyceride | 2,00 | 2,00 |
| Glyceryl Stearate | 1,70 | 1,70 |
| Hydrogenated Coco-Glycerides | 1,00 | 1,00 |
| Ethylhexyl Cocoate | 2,00 | 2,00 |
| Dimethicone | 0,30 | 0,30 |
| Sodium Stearoyl Glutamate | 0,50 | 0,50 |
| Silica Dimethyl Silylate | 0,30 | 0,30 |
| Tapioca Starch + Aqua | 1,00 | 1,00 |
| Parfum | 0,50 | 0,50 |
| Glycerin | 0,90 | 0,90 |
| Sodium Hydroxide | 0,25 | 0,25 |
| Phenoxyethanol | 0,60 | 0,60 |
| Methylparaben | 0,30 | 0,30 |
| Acrylates/C10-30 Alkyl Acrylate Crosspolymer | 0,20 | 0,20 |
| Xanthan Gummi | 0,50 | 0,50 |
| Alcohol Denat. | 8,00 | 8,00 |
| Homosalate | 9,00 | 9,00 |
| Ethylhexyl Salicylate | 4,50 | 4,50 |
| Bis-Ethylhexyloxyphenol Methoxyphenyl Triazine | 1,00 | 1,00 |
| Butyl Methoxydibenzoylmethane | 4,50 | 4,50 |
| Octocrylene | 9,00 | 9,00 |
| Phenylbenzimidazole Sulfonic Acid | 1,00 | 1,00 |
| Aqua | ad. 100 | ad. 100 |
| | | |
| Reduktion b* [%] | -19 | -38 |

Die Ergebnisse zeigen eine eindeutige Fleckenverminderung durch den Einsatz von Komplexbildnern im Vergleich zur Formulierung ohne die erfindungsgemäßen Komplexbildner (Beispiel 1).

**Tabelle 1: getestete Zubereitungen und deren Gelbwert-Reduktion von Flecken; b*-Wert [%]**

| **INCI** | **Beispiel [%]** | |
|---|---|---|
| | **3** | **4** |
| Tetrasodium Iminodisuccinate | 0,60 | 0,60 |
| Diethylentriamin-penta(methylenphosphonsäure) | | 0,50 |
| Trisodium EDTA | 0,30 | 0,30 |
| Cetearyl Alcohol | 0,50 | 0,50 |
| Glyceryl Stearate | 1,70 | 1,70 |
| Hydrogenated Coco-Glycerides | 1,00 | 1,00 |
| Dimethicone | 0,30 | 0,30 |
| Sodium Stearoyl Glutamate | 0,50 | 0,50 |
| Silica Dimethyl Silylate | 0,50 | 0,50 |
| Tapioca Starch + Aqua | 1,00 | 1,00 |
| Parfum | 0,60 | 0,60 |
| Glycerin | 0,90 | 0,90 |
| Sodium Hydroxide | 0,25 | 0,25 |
| Phenoxyethanol | 0,60 | 0,60 |
| Methylparaben | 0,30 | 0,30 |
| Acrylates/C10-30 Alkyl Acrylate Crosspolymer | 0,20 | 0,20 |
| Xanthan Gummi | 0,50 | 0,50 |
| Alcohol Denat. | 6,00 | 6,00 |
| Homosalate | 9,00 | 9,00 |
| Ethylhexyl Salicylate | 4,50 | 4,50 |
| Bis-Ethylhexyloxyphenol Methoxyphenyl Triazine | 3,50 | 3,50 |
| Butyl Methoxydibenzoylmethane | 4,50 | 4,50 |
| Octocrylene | 8,00 | 8,00 |
| Phenylbenzimidazole Sulfonic Acid | 1,00 | 1,00 |
| Aqua | ad. 100 | ad. 100 |
| | | |
| Reduktion b* [%] | -22 | -29 |

Die Ergebnisse zeigen eine eindeutige Fleckenverminderung durch den Einsatz von Komplexbildnern im Vergleich zur Formulierung ohne die erfindungsgemäßen Komplexbildner (Beispiel 4).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen

| **INCI** | **Beispiel [%]** | | | | |
|---|---|---|---|---|---|
| | **1** | **2** | **3** | **4** | **5** |
| Tetranatrium im inodisuccinate | 0,50 | | | | 0,50 |
| Pentakalium-ATMP-oxid | | 0,50 | | | |
| Diethylentriam in-penta(methylenphosphonsäure) | | | 0,50 | | |
| Natriumpolyphosphat | | | | 0,50 | |
| Tetranatrium Pyrophosphat | 0,50 | | | | |
| Natriumdiethylenetriaminepentamethylenephosphonat | | 0,50 | | | |
| Natrium EDTMP | | | 0,50 | | |
| Diethylentriaminpentamethylenphosphonsäure/ Salzsäure | | | | 0,50 | |
| Ethylendiamin-tetramethylenphosphonsäure | | | | | 0,50 |
| Trisodium EDTA | 0,20 | 0,50 | 0,50 | 0,20 | 0,20 |
| Butyl methoxydibenzoylmethane | 5,00 | 3,00 | 3,00 | 5,00 | 4,50 |
| Diethylamino Hydroxybenzoyl Hexyl Benzoate | | | | 0,5 | |
| Bis-Ethylhexyloxyphenol Methoxyphenyl Triazine | 3,50 | 3,50 | 0,50 | 0,50 | 3,50 |
| Phenylbenzimidazole Sulfonic Acid | | 1,00 | 1,00 | 1,00 | 1,00 |
| Ethylhexyl Salicylate | | 5,00 | 5,00 | 5,00 | |
| Titanium Dioxide | 3,00 | 2,00 | | | 3,00 |
| Trimethoxycaprylylsilane | 0,20 | 0,20 | | | 0,20 |
| Octocrylene | 10,00 | 10,00 | 10,00 | 10,00 | 9,00 |
| Homosalate | | 10,00 | 10,00 | 10,00 | 9,50 |
| Cetearyl Alcohol | | 1,00 | 0,50 | | |
| Xanthan Gum | 0,40 | 0,40 | 0,40 | | 0,40 |
| Acrylates/C10-30 Alkyl Acrylate Crosspolymer | 0,30 | 0,05 | 0,20 | 0,40 | 0,30 |
| Alcohol Denat. | 5,00 | 4,00 | 6,00 | 6,00 | 5,00 |
| Methylparaben | 0,30 | 0,30 | 0,30 | 0,30 | 0,30 |
| Phenoxyethanol | 0,60 | 0,60 | 0,60 | 0,60 | 0,60 |
| Citric Acid | 0,30 | | | | 0,30 |
| Sodium Citrate | 0,10 | | | | 0,10 |
| Sodium Hydroxide | 0,20 | 0,40 | 0,50 | 0,70 | 0,20 |
| Glycerin | 3,00 | 9,00 | 3,00 | 9,00 | 3,00 |
| Parfum | | 0,40 | 0,60 | 0,30 | |
| VP/Hexadecene Copolymer | 0,50 | 0,50 | | 0,50 | 0,50 |
| Silica Dimethyl Silylate | | 0,50 | 0,50 | | |
| Sodium Cetearyl Sulfate | | 0,15 | | | |
| Glyceryl Stearate SE | | 1,00 | | | |
| Glyceryl Stearate Citrate | 2,00 | | | | 2,00 |
| Ceteareth-20 | | | | 1,00 | |
| Sodium Stearoyl Glutamate | | | 0,40 | | |
| Glyceryl Stearate | | | 1,00 | | |
| Hydrogenated Coco-Glycerides | 1,00 | | 1,00 | | 1,00 |
| C12-15 Alkyl Benzoate | 5,00 | | | | 5,00 |
| Myristyl Myristate | 1,00 | 1,00 | | | 1,00 |
| Stearyl Alcohol | 0,50 | | | | 0,50 |
| C18-36 Acid Triglyceride | | | | 0,50 | |
| C18-38 Alkyl Hydroxystearoyl Stearate | | 0,50 | | | |
| Isopropyl Stearate | 2,00 | | | | 2,00 |
| Butylene Glycol Dicaprylate/Dicaprate | 5,00 | | | 3,00 | 5,00 |
| Aqua | ad 100 | ad 100 | ad 100 | ad 100 | ad 100 |

## Patentansprüche

1. Verfahren zur Erleichterung der Auswaschbarkeit von UV-Lichtschutzfilter enthaltenden kosmetischen Zubereitungen aus Textilien, wobei dem Kosmetikum ein oder mehrere Komplexbildner aus der Gruppe
- 1-Hydroxyethan-(1,1-diphosphonsäure)/HEDP
- Aminotrimethylenphosphonsäure/ATMP
- Diethylentriaminpenta(methylenphosphsonsäure)/DTPMP
- Ethylendiamintetra(methylenphosphonsäure/EDTMP
- Phosphonobutan-tricarbonsäure/PBTC
- Natriumpolyphosphat
- Tetranstriumpyrophosphat
und/oder deren Alkalisalze zugesetzt werden, **dadurch gekennzeichnet, dass** die Zubereitung 0,1 bis 3,0 Gewiehts-%, bezogen auf das Gesamtgewicht der Zubereitung, an Komplexbildnem enthält.

2. Vertahren zur Reduzierung der durch UV-Lichtschutzfilter enthaltenden kosmetischen Zubereitungen hervorgerufenen Textilverfleckung, wobei dem Kosmetikum ein oder mehrere Komplexbildner aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPP
- Ethylendiamintetra(methylenphosphonsäure/ EDTMP
- Phosphonobutan-tricarbonsäure/ PBTC
- Natriumpolyphosphat
- Tetranatriumpyrophosphat
und/oder deren Alkalisalze zugesetzt werden, **dadurch gekennzeichnet, dass** die Zubereitung 0,1 bis 3,0 Gewichts-%, bezogen auf das Gesamtgewicht der Zubereitung,an Komplexbildnem enthält.

3. Verwendung ; von Komplexbildnem aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Natriumpolyphosphat
- Tetranatriumpyrophosphat
und/oder deren Alkalisalze,
in UV-Lichtschutzfilter enthaltenden kosmetischen Zubereitungen zur Erleichterung der Auswaschbarkeit der UV-Lichtshutzfilter aus mit den Zubereitungen kontaminierten Textilien, **dadurch gekennzeichnet, dass** die Zubereitung 0,1 bis 3,0 Gewichts-%, bezogen auf das Gesamtgewicht der Zubereitung, an Komplexbildnem enthält.

4. Verwendung von Komplexbildnem aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Natriumpolyphosphat
- Tetranatriumpyrophosphat
und/oder deren Alkalisalze
in UV-Lichtschutzfilter enthaltenden kosmetischen Zubereitungen zur Reduzierung der durch die Zubereitung hervorgerufene Textilverfleckung, **dadurch gekennzeichnet, dass** die Zubereitung 0,1 bis 3,0 Gewichts-%, bezogen auf das Gesamtgewicht der Zubereitung, an Komplexbildnem enthält.

5. Verfahren oder Verwendung nach einem der vorhergehenden Ansprüche, **dadurch gekennzeichnet, dass** als Komplexbildner eine oder mehrere der Verbindungen aus der Gruppe
- Diethylentriaminpenta(methylenphosphonsäure)/ DTPMP
- Ethylendiamintetra(methylenphosphonsäure/ EDTMP
- Aminotrimethylenphosphonsäure/ ATMP
- Phosphonobutan-tricarbonsäure/ PBTC
- Natriumpolyphosphat
- Tetranatriumpyrophosphat.
und/oder deren Alkalisalze und/oder deren Amin-N-Oxide eingesetzt werden.

6. Verfahren oder Verwendung nach einem der vorhergehenden Ansprüche, **dadurch gekennzeichnet, dass** die kosmetische Zubereitung einen oder mehrere UV-Filter gewählt aus der Gruppe der Verbindungen 4-(tert-Butyl)-4'-methoxydibenzoylmethan (INCI Butyl Methoxydibenzoylmethane), Hexyl 2-[4-(diethylamino)-2-hydroxybenzoyl]benzoate (INCI Diethylamino Hydroxybenzoyl Hexyl Benzoate) und 2,4-Bis-{[4-(2-ethyl-hexyloxy)-2-hydroxy]-phenyl]-6-(4-methoxyphenyl)-1,3,5-triazin (INCI Bis-Ethylhexyloxyphenol Methoxyphenyl Triazine) enthält.

7. Verfahren oder Verwendung nach Anspruch 9, **dadurch gekennzeichnet, dass** das Gewichtsverhältnis der gesamtmenge aus 4-(tert-Butyl)-4'-methoxydibenzoylmethan (INCI Butyl Methoxydibenzoylmethane), INCI Butyl Methoxydibenzoylmethane), Hexyl 2-[4-(diethylamino)-2-hydroxybenzoyl]behzoate (INCI Dierhylamino Hydroxybenzoyl Hexyl Benzoate) und 2,4-Bis-{[4-(2-ethyl-hexyloxy)-2-hydrexy]-phenyl}-6-(4-methoxyphenyl)-1,3,5-triazin (INCI Bis-Ethylhexyloxyphenol Methoxypoenyl Triazine) zur Gesamtmenge an Komplexbildnern von 5:1 bis 5:3 beträgt

8. Verfahren oder Verwendung nach einem der vorhergehenden Ansprüche, **dadurch gekennzeichnet, dass** die kosmetische Zubereltung einen oder mehrere UV-Filterenthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Mathylbenzyliden)campher; 3-Benzylidencampher, Etbylhexylsallcylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethythexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-:(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,4-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

9. Verfahren oder Verwahdung nach einem der vorhergehenden Ansprüche, dadurch gekennzelchnet, dass die Zubereitung in Form einer Öl-in-Wasser-Emulsion (O/W-Emulsion) vorliegt.

10. Verfahren oder Verwendung nach einem der vorhergebenden Ansprüche, **dadurch gekennzeichnet, dass** die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat Natriumstearoylglutamat, Polyglyceryl-3-metylglucosedistearat, Stearinsäure, Kaliumcetylphosphat, enthält.

11. Verfahren oder Verwendung nach einem der vorhergehenden Ansprüche, **dadurch gekennzeichnet, dass** die Zubereitung Acrylat/C10-30 Alkylacrylat-Crosspolymer enthält.

12. Verfahren oder Verwendung nach einem der vorhergehenden Ansprüche, **dadurch gekennzeichnet, dass** die Zubereitung Vinylpyrrolidon/Hexadecen Copolymer enthält

13. Verfahren oder Verwendung nach einem der vorhergehenden Ansprüche, **dadurch gekennzeichnet, dass** die Zubereitung Dialkylcarbonat, Dialkyladipat und/oder Dialkylglutarat enthält.

14. Verfahren oder Verwendung nach einem der vorhergehenden Ansprüche, **dadurch gekennzeichnet, dass** die Zubereitung Di-n-Octylcarbonat (INCI Dicaprylyl Carbonate) und/oder Di-n-Butyladipat (INCI Dibutyl Adipate) enthält.

## Claims

1. Method for facilitating the ability of cosmetic preparations comprising UV photoprotective filters to be washed out from textiles, wherein one or more complexing agents from the group comprising
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- sodium polyphosphate
- tetrasodium pyrophosphate
and/or alkali metal salts thereof, a
re added to the cosmetic **characterized in that** the preparation comprises 0.1 to 3.0% by weight of complexing agents, based on the total weight of the preparation.

2. Method for reducing the textile staining caused by cosmetic preparations comprising UV photoprotective filters, wherein one or more complexing agents from the group comprising
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- sodium polyphosphate
- tetrasodium pyrophosphate
and/or alkali metal salts thereof, a
re added to the cosmetic **characterized in that** the preparation comprises 0.1 to 3.0% by weight of complexing agents, based on the total weight of the preparation.

3. Use of complexing agents from the group comprising
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- sodium polyphosphate
- tetrasodium pyrophosphate
and/or alkali metal salts thereof, in
cosmetic preparations comprising UV photoprotective filters for facilitating the ability of UV photoprotective filters to be washed out from textiles contaminated with the preparations, **characterized in that** the preparation comprises 0.1 to 3.0% by weight of complexing agents, based on the total weight of the preparation.

4. Use of complexing agents from the group comprising
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- sodium polyphosphate
- tetrasodium pyrophosphate
and/or alkali metal salts thereof,
in cosmetic preparations comprising UV photoprotective filters for reducing the textile staining caused by the preparation, **characterized in that** the preparation comprises 0.1 to 3.0% by weight of complexing agents, based on the total weight of the preparation.

5. Method or use according to any of the preceding claims, **characterized in that** the complexing agents used are one or more of the compounds from the group comprising
- diethylenetriaminepenta(methylenephosphonic acid)/ DTPMP
- ethylenediaminetetra(methylenephosphonic acid)/ EDTMP
- aminotrimethylenephosphonic acid/ ATMP
- phosphonobutanetricarboxylic acid/ PBTC
- sodium polyphosphate
- tetrasodium pyrophosphate
and/or alkali metal salts thereof and/or amine-N-oxides thereof.

6. Method or use according to any of the preceding claims, **characterized in that** the cosmetic preparation comprises one or more UV filters selected from the group of the compounds 4-(tert-butyl)-4'-methoxydibenzoylmethane (INCI Butyl Methoxydibenzoylmethane), hexyl 2-[4-(diethylamino)-2-hydroxybenzoyl]benzoate (INCI Diethylamino Hydroxybenzoyl Hexyl Benzoate) and 2,4-bis-{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine).

7. Method or use according to Claim 9, **characterized in that** the ratio by weight of the total amount of 4-(tert-butyl)-4'-methoxydibenzoylmethane (INCI Butyl Methoxydibenzoylmethane), INCI Butyl Methoxydibenzoylmethane), hexyl 2-[4-(diethylamino)-2-hydroxybenzoyl]benzoate (INCI Diethylamino Hydroxybenzoyl Hexyl Benzoate) and 2,4-bis{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine) to the total amount of complexing agents is from 5:1 to 5:3.

8. Method or use according to any of the preceding claims, **characterized in that** the cosmetic preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

9. Method or use according to any of the preceding claims, **characterized in that** the preparation is in the form of an oil-in-water emulsion (O/W emulsion).

10. Method or use according to any of the preceding claims, **characterized in that** the preparation comprises one or more emulsifiers selected from the group of compounds comprising glyceryl stearate citrate, cetearyl alcohol sodium cetearyl sulfate + glyceryl stearate, cetearyl sulfosuccinate, sodium stearoyl glutamate, polyglyceryl-3 methylglucose distearate, stearic acid, potassium cetyl phosphate.

11. Method or use according to any of the preceding claims, **characterized in that** the preparation comprises acrylate/C10-30 alkyl acrylate crosspolymer.

12. Method or use according to any of the preceding claims, **characterized in that** the preparation comprises vinylpyrrolidone/hexadecane copolymer.

13. Method or use according to any of the preceding claims, **characterized in that** the preparation comprises dialkyl carbonate, dialkyl adipate and/or dialkyl glutarate.

14. Method or use according to any of the preceding claims, **characterized in that** the preparation comprises di-n-octyl carbonate (INCI Dicaprylyl Carbonate) and/or di-n-butyl adipate (INCI Dibutyl Adipate).

## Revendications

1. Procédé visant à faciliter la rinçabilité de préparations cosmétiques contenant des filtres de protection contre la lumière UV à partir de textiles, un ou plusieurs complexants du groupe constitué par :
- l'acide 1-hydroxyéthane-(1,1-diphosphonique)/HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique/ PBTC,
- le polyphosphate de sodium,
- le pyrophosphate de tétrasodium,
et/ou leurs sels alcalins étant ajoutés au cosmétique, **caractérisé en ce que** la préparation contient 0,1 à 3,0 % en poids, par rapport au poids total de la préparation, de complexants.

2. Procédé visant à réduire les taches sur des textiles causées par des préparations cosmétiques contenant des filtres de protection contre la lumière UV, un ou plusieurs complexants du groupe constitué par :
- l'acide 1-hydroxyéthane-(1,1-diphosphonique)/ HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique/ PBTC,
- le polyphosphate de sodium,
- le pyrophosphate de tétrasodium,
et/ou leurs sels alcalins étant ajoutés au cosmétique, **caractérisé en ce que** la préparation contient 0,1 à 3,0 % en poids, par rapport au poids total de la préparation, de complexants.

3. Utilisation de complexants du groupe constitué par :
- l'acide 1-hydroxyéthane-(1,1-diphosphonique)/ HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique/ PBTC,
- le polyphosphate de sodium,
- le pyrophosphate de tétrasodium,
et/ou leurs sels alcalins,
dans des préparations cosmétiques contenant des filtres de protection contre la lumière UV pour faciliter la rinçabilité des filtres de protection contre la lumière UV à partir de textiles contaminés avec les préparations, **caractérisée en ce que** la préparation contient 0,1 à 3,0 % en poids, par rapport au poids total de la préparation, de complexants.

4. Utilisation de complexants du groupe constitué par :
- l'acide 1-hydroxyéthane-(1,1-diphosphonique)/ HEDP,
- l'acide aminotriméthylène-phosphonique/ ATMP,
- l'acide diéthylène-triamine-penta(méthylène-phosphonique)/ DTPMP,
- l'acide éthylène-diamine-tétra(méthylène-phosphonique)/ EDTMP,
- l'acide phosphonobutane-tricarboxylique/ PBTC,
- le polyphosphate de sodium,
- le pyrophosphate de tétrasodium,
et/ou leurs sels alcalins,
dans des préparations cosmétiques contenant des filtres de protection contre la lumière UV pour réduire les taches sur des textiles causées par la préparation, **caractérisée en ce que** la préparation contient 0,1 à 3,0 % en poids, par rapport au poids total de la préparation, de complexants.

5. Procédé ou utilisation selon l'une quelconque des revendications précédentes, caractérisé ou **caractérisée en ce qu'**un ou plusieurs des composés du groupe constitué par :
- l'acide diéthylène-triamine-penta(méthylène-phosphonique)/ DTPMP,
- l'acide éthylène-diamine-tétra(méthylène-phosphonique)/ EDTMP,
- l'acide aminotriméthylène-phosphonique/ ATMP,
- l'acide phosphonobutane-tricarboxylique/ PBTC,
- le polyphosphate de sodium,
- le pyrophosphate de tétrasodium,
et/ou leurs sels alcalins et/ou leurs amine-N-oxydes sont utilisés en tant que complexants.

6. Procédé ou utilisation selon l'une quelconque des revendications précédentes, caractérisé ou **caractérisée en ce que** la préparation cosmétique contient un ou plusieurs filtres UV choisis dans le groupe de composés constitué par le 4-(tert.-butyl)-4'-méthoxydibenzoylméthane (INCI : Butyl Methoxydibenzoylmethane), le 2-[4-(diéthylamino)-2-hydroxybenzoyl]benzoate d'hexyle (INCI : Diethylamino Hydroxybenzoyl Hexyl Benzoate) et la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

7. Procédé ou utilisation selon la revendication 9, caractérisé ou **caractérisée en ce que** le rapport en poids entre la quantité totale de 4-(tert.-butyl)-4'-méthoxydibenzoylméthane (INCI : Butyl Methoxydibenzoylmethane), INCI : Butyl Methoxydibenzoylmethane), de 2-[4-(diéthylamino)-2-hydroxybenzoyl]benzoate d'hexyle (INCI : Diethylamino Hydroxybenzoyl Hexyl Benzoate) et de 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine), et la quantité totale de complexants est de 5:1 à 5:3.

8. Procédé ou utilisation selon l'une quelconque des revendications précédentes, caractérisé ou **caractérisée en ce que** la préparation cosmétique contient un ou plusieurs filtres UV, qui sont choisis dans le groupe de composés constitué par l'acide 2-phénylbenzimidazole-5-sulfonique et/ou ses sels, les sels de l'acide phénylène-1,4-bis-(2-benzimidazyl)-3,3'-5,5'-tétrasulfonique, le 1,4-di(2-oxo-10-sulfo-3-bornylidène-méthyl)-benzène et ses sels, les sels de l'acide 4-(2-oxo-3-bornylidène-méthyl)benzène-sulfonique, les sels de l'acide 2-méthyl-5-(2-oxo-3-bornylidène-méthyl)sulfonique, le 2,2'-méthylène-bis-(6-(2H-benzotriazol-2-yl)-4-(1,1,3,3-tétraméthylbutyl)-phénol), le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol, le 3-(4-méthylbenzylidène)camphre, le 3-benzylidène-camphre, le salicylate d'éthylhexyle, l'acide téréphtalidène-dicamphre-sulfonique, l'acrylate de 2-éthylhexyl-2-cyano-3,3-diphényle, l'ester 2-éthylhexylique de l'acide 4-(diméthylamino)-benzoïque, l'ester amylique de l'acide 4-(diméthylamino)benzoïque, l'ester di(2-éthylhexylique) de l'acide 4-méthoxybenzalmalonique, l'ester 2-éthylhexylique de l'acide 4-méthoxycinnamique, l'ester isoamylique de l'acide 4-méthoxycinnamique, la 2-hydroxy-4-méthoxybenzophénone, la 2-hydroxy-4-méthoxy-4'-méthylbenzophénone, la 2,2'-dihydroxy-4-méthoxybenzophénone, le salicylate d'homomenthyle, le 2-hydroxybenzoate de 2-éthylhexyle, le benzalmalonate de diméthicodiéthyle, le copolymère de 3-(4-(2,2-bis-éthoxycarbonylvinyl)-phénoxy)propényl)-méthoxysiloxane/diméthylsiloxane, l'ester hexylique de l'acide 2-(4'-diéthylamino-2'-hydroxybenzoyl)-benzoïque, la dioctylbutylamidotriazone (INCI: Diethylhexyl-Butamidotriazone), la 2,4-bis-[5-1-(diméthylpropyl)benzoxazol-2-yl-(4-phényl)-imino]-6-(2-éthylhexyl)-imino-1,3,5-triazine de n° CAS 288254-16-0), l'ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI: Ethylhexyl Triazone), la 2,4,6-tribiphényl-4-yl-1,3,5-triazine, la mérocyanine, le dioxyde de titane, l'oxyde de zinc.

9. Procédé ou utilisation selon l'une quelconque des revendications précédentes, caractérisé ou **caractérisée en ce que** la préparation se présente sous la forme d'une émulsion huile dans eau (émulsion H/E).

10. Procédé ou utilisation selon l'une quelconque des revendications précédentes, caractérisé ou **caractérisée en ce que** la préparation contient un ou plusieurs émulsifiants choisis dans le groupe de composés constitué par le stéarate-citrate de glycéryle, l'alcool cétéarylique, le sulfate de cétéaryle sodium + le stéarate de glycéryle, le sulfosuccinate de cétéaryle, le glutamate de stéaroyle sodium, le distéarate de polyglycéryl-3-méthylglucose, l'acide stéarique, le phosphate de cétyle potassium.

11. Procédé ou utilisation selon l'une quelconque des revendications précédentes, caractérisé ou **caractérisée en ce que** la préparation contient de l'Acrylates/C10-30 Alkyl Acrylate Crosspolymer.

12. Procédé ou utilisation selon l'une quelconque des revendications précédentes, caractérisé ou **caractérisée en ce que** la préparation contient un copolymère de vinylpyrrolidone/hexadécène.

13. Procédé ou utilisation selon l'une quelconque des revendications précédentes, caractérisé ou **caractérisée en ce que** la préparation contient du carbonate de dialkyle, de l'adipate de dialkyle et/ou du glutarate de dialkyle.

14. Procédé ou utilisation selon l'une quelconque des revendications précédentes, caractérisé ou **caractérisée en ce que** la préparation contient du carbonate de di-n-octyle (INCI : Dicaprylyl Carbonate) et/ou de l'adipate de di-n-butyle (INCI : Dibutyl Adipate).
